# EUROPEAN PATENT SPECIFICATION

(11) **EP 3 338 903 B1**
(45) Date of publication and mention of the grant of the patent: **06.11.2019**
(21) Application number: 16894162.3
(22) Date of filing: 29.09.2016
(51) Int. Cl.: B08B 3/12, B08B 3/10, B08B 3/08, A61L 2/07, A61L 2/025

(54) **CLEANSING AND STERILIZING DEVICE USING PULSE VACUUM AND CLEANSING METHOD**
REINIGUNGS- UND STERILISIERVORRICHTUNG MIT PULSVAKUUM UND REINIGUNGSVERFAHREN
DISPOSITIF DE NETTOYAGE ET DE STÉRILISATION AU MOYEN DE VIDE À IMPULSIONS ET PROCÉDÉ DE NETTOYAGE

(30) Priority: 16.03.2016 CN 201610149889
(43) Date of publication of application: 27.06.2018
(73) Proprietor: Lianyungang Youyuan Medical Equipment Manufacturing Co., Ltd., Lianyungang, Jiangsu 222000 (CN)
(72) Inventor: ZHANG, Jianbin, Lianyungang Jiangsu 222000 (CN); LV, Xinlei, Jiangsu 222000 (CN)
(74) Representative: Sun, Yiming
(86) International application number: PCT/CN2016/100968
(87) International publication number: WO 2017/157003

(56) References cited:
- CN-A- 1 305 848
- CN-A- 101 722 160
- CN-A- 102 333 600
- CN-A- 103 878 153
- CN-A- 105 562 391
- CN-U- 202 141 288
- CN-U- 205 413 759
- CN-Y- 201 423 361
- JP-A- H05 131 019
- JP-A- 2003 033 737

## Description

### TECHNICAL FIELD OF THE INVENTION

The present invention relates to the technical field of sterilizing devices, and more particularly, to a cleansing and sterilizing device using pulse vacuum and a cleansing method thereof.

### BACKGROUND OF THE INVENTION

A cleansing machine is a device used for cleansing specific instruments or equipment, and is capable of effectively prolonging the functional life of the instruments or equipment while satisfying related cleansing requirements during use. In the prior art, a traditional medical instrument cleansing device usually adopts a spray-head cleansing technology. As a result, the spraying nozzle needs to be adjusted time after time during the cleansing process of tubular articles. When cleansing various tubular articles or surgical instruments such as a dental hand-piece, a dressing bowl, a precise apparatus or a scissor, articles cannot be simultaneously placed onto a cleansing plate before specialized brackets are ready for them. Such a process is complicated and laborious. Meanwhile, as can be easily contaminated, the spraying hose and the nozzle connector need to be cleansed frequently, resulting in a low cleansing efficiency and a heavy labor burden.

Chinese patent 201510562538.6 (filed on Sep.7, 2015, approved and published as CN 105170581 A on Dec.23, 2015) discloses an automatic pulse pipeline cleansing device, which comprises a central controller, a water-gas pulse generator, a liquid pump and a cleaning main body frame. The central controller is electrically connected to the water-gas mixed pulse generator and the liquid pump, and the liquid pump is electrically connected to the water-gas mixed pulse generator. Compared with the prior art, the periphery of the cleansing main body frame of the aforesaid invention is provided with an automatic walking unit. As the walking unit can freely walk within the pipeline, the water-gas pulse can be sprayed into the depths or bending portions of the pipeline. Thus, the pipeline can be thoroughly cleansed, achieving an ideal effect of the water-gas mixed pulse cleansing.

Chinese patent 201310029690.9 (filed on Jan.25, 2013, approved and published as CN 103071651 A on May 1, 2015) also discloses a method that uses spraying and pulse air to cleanse a transformer cooler, which aims to solve the technical problems relating to a poor cleansing effect, a long cleansing time and a high labor intensity in the prior art, and protect the equipment from being damaged during the cleansing process. The high-pressure cleansing solution of the aforesaid invention can be atomized and sprayed onto the transformer cooler through a cleansing solution output pipe and an atomizing nozzle, thereby softening the solid or flocculent dirt on the outer surface of the transformer cooler. After absorbing air, the air compressor compresses the air into a high-pressure air, which is transmitted into the air pulse mechanism through the high-pressure air hose. The air pulse mechanism converts the high pressure air into a high-pressure pulse air, which is used to cleanse the transformer cooler through the pulse air hose and the air cap. The aforesaid invention is merely used for cleansing transformer coolers.

Furthermore, Chinese patent 201410283954.8 (filed on Jun.24, 2014, approved and published as CN 104048556 A on Sep 17, 2014) discloses a pulse circulation cleansing device for a heat exchanger, which mainly comprises a gas buffer tank, a pulse generator, a gas-liquid mixer and a switching valve. The gas buffer tank is used for providing compressed air. The gas inlet of the pulse generator is connected to the gas outlet of the gas buffer tank. The gas-liquid mixer is provided with a gas inlet, a liquid inlet and a gas-liquid outlet. The liquid inlet is connected to the outlet of a cleansing pump. The switching valve is provided with a water inlet, a discharge port, a circulating water supply port and a circulating water return port, and the water inlet is connected to the gas-liquid outlet. The aforesaid invention also provides a method for cleansing the heat exchanger. The cleansing device combines technologies such as gas-liquid pulse cleansing, circulation cleansing and chemical circulation cleansing, and is simple, widely-applicable, highly-efficient and cost-effective. The cleansing device can be used to continuously cleanse various heat exchangers.

The aforesaid technical solutions or the combination of them do not affect the novelty and the creativity of the present invention.

The document CN 102333600 A discloses a sterilizing and cleansing device in accordance with the preamble of claim 1, and a cleansing method.

### SUMMARY OF THE INVENTION

As the traditional cleansing device usually adopts a spray-head cleansing technology, the spraying nozzle needs to be adjusted time after time during the cleansing process of tubular articles. Meanwhile, various tubular articles or surgical instruments cannot be simultaneously placed onto a cleansing plate before specialized brackets are ready. Such a process is complicated and laborious. As can be easily contaminated, the spraying hose and the nozzle connector need to be cleansed frequently, resulting in a low cleansing efficiency and a heavy labor burden. The purpose of the present invention is to solve the shortcomings in the prior art by providing a cleansing and sterilizing device using pulse vacuum and a cleansing method thereof.

To achieve the above purpose, the present invention adopts the technical solution in accordance with claim 1:
A cleansing and sterilizing device using pulse vacuum comprising a sterilizing and cleansing device 1, an air purification device 2, a water purification device 3 and a pipeline 4; the sterilizing and cleansing device 1 is connected to the air purification device 2 and the water purification device 3; the sterilizing and cleansing device 1 further comprises a cleansing basin 101, a steam coil 102, a cleansing basin heating pipe 103, an ultrasonic sterilizing device 104, a water discharge valve 105, a steam valve 106, a manually-operated valve 107, a detergent valve 108, a detergent compartment 109, a vacuum pump valve 110, a vacuum pump 111 and an aluminum plate 112; the cleansing basin 101 is a hollow cavity structure, and a stream coil 102 is arranged in the interior of the side wall and the top portion of the cleansing basin; the inner surface of the cleansing basin 101 is provided with an aluminum plate 112, and the bottom surface of the cleansing basin 101 is connected to the cleansing basin heating pipe 103 and the ultrasonic sterilizing device 104; the cleansing basin 101 is connected to the water discharge valve 105 through the pipeline 4, and the lower portion of the cleansing basin 101 is connected to the detergent compartment 109 through the pipeline 4. The detergent valve 108 is arranged on the pipeline 4 between the cleansing basin 101 and the detergent compartment 109. The upper portion of the cleansing basin 101 is connected to the vacuum pump 111 through the pipeline 4, and the vacuum pump valve 110 is arranged on the pipeline 4 between the cleansing basin 101 and the vacuum pump 111. The steam valve 106 and the manually-operated valve 107 are arranged on the steam coil 102.

In another aspect of the present invention, the air purification device 2 further comprises a bottom air inlet coil pipe 201, a bottom air inlet valve 202, a plasma sterilizer 203, an air filter 204 and a top portion air return valve 205. The bottom surface of the cleansing basin 101 is connected to the bottom air inlet coil pipe 201, and the bottom air inlet coil pipe 201 is connected to the plasma sterilizer 203 through the pipeline 4. The bottom air inlet valve is arranged on the pipeline 4 between the bottom air inlet coil pipe 201 and the plasma sterilizer 203. The plasma sterilizer 203 is connected to the air filter 204 and the top portion of the cleansing basin 101 through the pipeline 4. The top portion air return valve 205 is arranged on the pipeline 4 between the plasma sterilizer 203 and the top portion of the cleansing basin 101.

In another aspect of the present invention, the water purification device 3 comprises a water storage tank 301, a water storage tank heating pipe 302, a water storage tank outlet valve 303, a water storage tank purified water inlet valve 304, a cleansing basin purified water inlet valve 305 and a purified water tank 306. The lower portion of the cleansing basin 101 is connected to the water storage tank 301 and the purified water tank 306 through the pipeline 4. The water storage tank outlet valve 303 is arranged on the pipeline 4 between the lower portion of the cleansing basin 101 and the water storage tank 301. The cleansing basin purified water inlet valve 305 is arranged on the pipeline 4 between the lower portion of the water cleansing basin 101 and the purified water tank 306. The water storage tank heating pipe 302 is arranged within the water storage tank 301, and the water storage tank 301 is connected to the purified water tank 306 through the pipeline 4. The water storage tank purified water inlet valve 304 is arranged on the pipeline 4 between the water storage tank 301 and the purified water tank 306.

A cleansing method in accordance with claim 4, using a cleansing and sterilizing device of the present invention, comprises the steps of:
Step 1: placing the instrument 5 into the cleansing basin 101, sealing the cleansing basin 101, and simultaneously adding the cleansing water and the detergent to prepare a cleansing solution; subsequently, heating the cleansing solution by the cleansing basin heating pipe 103; initiating the ultrasonic sterilizing device 104, thereby cleansing and sterilizing the instrument 5 via ultrasonic wave; soaking the instrument 5 in the cleansing solution for a period of time;
Step 2: turning on the vacuum pump valve 110 and initiating the vacuum pump 111; subsequently, exhausting the air in the cleansing basin 101, thereby enabling the cleansing solution to be boiled under a low-temperature vacuum condition; cleansing the instrument 5; subsequently, turning off the vacuum pump valve 110 and the vacuum pump 111 after the vacuum environment is formed;
Step 3: turning on the bottom air inlet valve 202, and initiating the air filter 204 and the plasma sterilizer 203; allowing the air to enter into the cleansing basin 101 through the bottom air inlet coil pipe after being filtered and sterilized by the air filter 204 and the plasma sterilizer 203; enabling the cleansing water to boil vigorously to cleanse the instrument 5;
Step 4: turning on the vacuum pump valve 110 and initiating the vacuum pump 111; subsequently, exhausting the air in the cleansing basin 101, thereby enabling the cleansing solution to be boiled under a low-temperature vacuum condition; cleansing the instrument 5; subsequently, turning off the vacuum pump valve 110 and the vacuum pump 111 after the vacuum environment is formed;
Step 5: turning on the top portion air return valve 205, and allowing the air to enter into the cleansing basin 101 through the top portion air return valve 205 after being filtered and sterilized by the air filter 204 and the plasma sterilizer 203; enabling the pressure in the cleansing basin 101 to increase rapidly so that the steam in the steam coil 102 can be compressed and liquefied accordingly; allowing the cleansing water to flow into the steam coil 102, thereby cleansing and sterilizing the instrument 5;
Step 6: repeating the above steps 2-5 for several times, thereby removing the dirt; turning on the water discharge valve 105 to discharge the cleansing water; turning on the cleansing basin purified water inlet valve 305, thereby allowing the purified water in the purified water tank 306 to enter into the cleansing basin 101; heating the purified water by the cleansing basin heating pipe 101; repeating the above steps 2-5, and turning on the water discharge valve 105 to discharge purified water;
Step 7: turning on the water storage tank outlet valve 303, thereby allowing the purified water that is heated by the water storage tank heating pipe 302 in the water storage tank 301 to enter into the cleansing basin 101; keeping the purified water at a temperature of 93°C and sterilizing the instrument 5; after repeating the above steps 2-5, turning on the water discharge valve to discharge the heated purified water;
Step 8: initiating the vacuum pump to vacuum the cleansing basin 101, thereby forming a vacuum environment therein; after the steam in the instrument 5 is exhausted, turning on the steam valve 106 along the decrease of the vacuum degree, thereby allowing the steam to flow through the steam coil 102 so that the heat energy can be transferred through the aluminum plate 112 to heat the cleansing basin 101; when the temperature and the vacuum degree simultaneously increase, re-vacuuming by the vacuum pump 111, thereby forming a vacuum environment; after repeating the above steps for several times, turning on the top portion air return valve to achieve an air return, thereby making the air pressure in the cleansing basin 101 return to the atmospheric pressure; finally, turning off the ultrasonic sterilizing device 104, and taking out the sterilized instrument 5.

Compared with the prior art, the present invention has the following advantages:
The cleansing and sterilizing device of the present invention uses a cleansing method combining a vacuum pulse and ultrasonic sterilization to perform cleansing and sterilization on medical instruments. The air purification device and the water purification device ensure that air and water entering into the sterilizing and cleansing device satisfy a cleansing requirement. The present invention replaces the traditional classified and manual cleansing by a fully automatic concentrated cleansing and sterilization, exhibits a favorable cleansing effect, achieves a high cleansing efficiency, and alleviates the labor burden of cleansing personnel.

### BRIEF DESCRIPTION OF THE DRAWINGS

To clearly expound the technical solution of the present invention, the drawings and embodiments are hereinafter combined to illustrate the present invention. Obviously, the drawings are merely some embodiments of the present invention and those skilled in the art can associate themselves with other drawings without paying creative labor.
Figure 1 is a structural diagram of the present invention;
Figure 2 is a structural diagram of the sterilizing and cleansing device of the present invention;
Figure 3 is a structural diagram of the air purification device of the present invention; and
Figure 4 is a structural diagram of the water purification device of the present invention.

### Marking Instructions of the Drawings:

1. Sterilizing and Cleansing Device; 101. Cleansing Basin; 102. Steam Coil; 103. Cleansing Basin Heating Pipe; 104. Ultrasonic Sterilizing Device; 105. Water Discharge Valve; 106. Steam Valve; 107. Manually-operated Valve; 108. Detergent Valve; 109. Detergent Compartment; 110. Vacuum Pump Valve; 111. Vacuum Pump; 112. Aluminum Plate;
2. Air Purification Device; 201. Bottom Air Inlet Coil Pipe; 202. Bottom Air Inlet Valve; 203. Plasma Sterilizer; 204. Air Filter; 205. Top Portion Air Return Valve;
3. Water Purification Device; 301. Water Storage Tank; 302. Water Storage Tank Heating Pipe; 303. Water Storage Tank Outlet Valve; 304. Water Storage Tank Purified Water Inlet Valve; 305. Cleansing Basin Purified Water Inlet Valve; 306. Purified Water Tank;
4. Pipeline;
5. Instrument.

### DETAILED DESCRIPTION OF THE INVENTION

Drawings and detailed embodiments are combined hereinafter to elaborate the technical principles of the present invention.

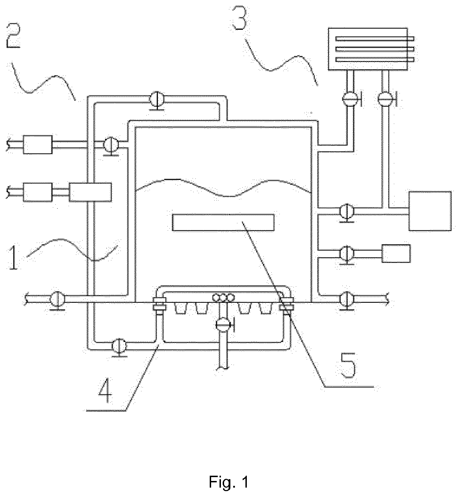

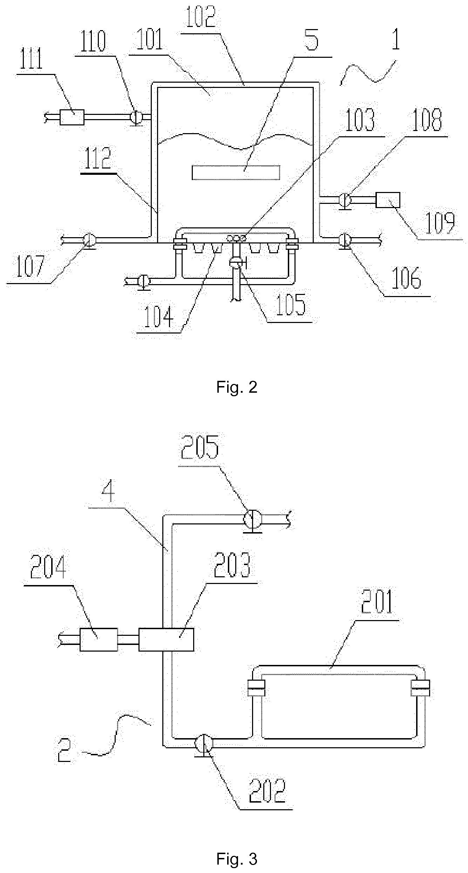

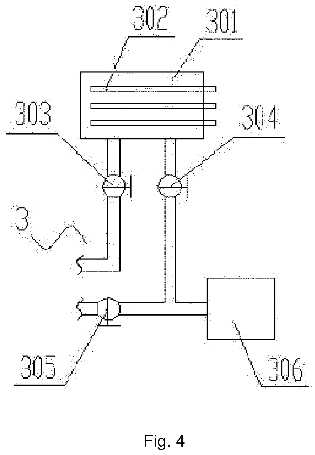

As shown in Figures 1-4, a cleansing and sterilizing device using pulse vacuum comprises a sterilizing and cleansing device 1, an air purification device 2, a water purification device 3 and a pipeline 4. The sterilizing and cleansing device 1 is connected to the air purification device 2 and the water purification device 3. The sterilizing and cleansing device 1 further comprises a cleansing basin 101, a steam coil 102, a cleansing basin heating pipe 103, an ultrasonic sterilizing device 104, a water discharge valve 105, a steam valve 106, a manually-operated valve 107, a detergent valve 108, a detergent compartment 109, a vacuum pump valve 110, a vacuum pump 111 and an aluminum plate 112. The cleansing basin 101 is a hollow cavity structure, and steam coils 102 are arranged in the interior of the side wall and the top portion of the cleansing basin. The inner surface of the cleansing basin 101 is provided with an aluminum plate 112, and the bottom surface of the cleansing basin 101 is connected to the cleansing basin heating pipe 103 and the ultrasonic sterilizing device 104. The cleansing basin 101 is connected to the water discharge valve 105 through the pipeline 4, and the lower portion of the cleansing basin 101 is connected to the detergent compartment 109 through the pipeline 4. The detergent valve 108 is arranged on the pipeline 4 between the cleansing basin 101 and the detergent compartment 109. The upper portion of the cleansing basin 101 is connected to the vacuum pump 111 through the pipeline 4, and the vacuum pump valve 110 is arranged on the pipeline 4 between the cleansing basin 101 and the vacuum pump 111. The steam valve 106 and the manually-operated valve 107 are arranged on the steam coil 102.

Preferably, the air purification device 2 further comprises a bottom air inlet coil pipe 201, a bottom air inlet valve 202, a plasma sterilizer 203, an air filter 204 and a top portion air return valve 205. The bottom surface of the cleansing basin 101 is connected to the bottom air inlet coil pipe 201, and the bottom air inlet coil pipe 201 is connected to the plasma sterilizer 203 through the pipeline 4. The bottom air inlet valve is arranged on the pipeline 4 between the bottom air inlet coil pipe 201 and the plasma sterilizer 203. The plasma sterilizer 203 is connected to the air filter 204 and the top portion of the cleansing basin 101 through the pipeline 4. The top portion air return valve 205 is arranged on the pipeline 4 between the plasma sterilizer 203 and the top portion of the cleansing basin 101.

Preferably, the water purification device 3 comprises a water storage tank 301, a water storage tank heating pipe 302, a water storage tank outlet valve 303, a water storage tank purified water inlet valve 304, a cleansing basin purified water inlet valve 305 and a purified water tank 306. The lower portion of the cleansing basin 101 is connected to the water storage tank 301 and the purified water tank 306 through the pipeline 4. The water storage tank outlet valve 303 is arranged on the pipeline 4 between the lower portion of the cleansing basin 101 and the water storage tank 301. The cleansing basin purified water inlet valve 305 is arranged on the pipeline 4 between the lower portion of the water cleansing basin 101 and the purified water tank 306. The water storage tank heating pipe 302 is arranged within the water storage tank 301, and the water storage tank 301 is connected to the purified water tank 306 through the pipeline 4. The water storage tank purified water inlet valve 304 is arranged on the pipeline 4 between the water storage tank 301 and the purified water tank 306.

A cleansing method in accordance with claim 4, using a cleansing and sterilizing device of the present invention, comprises the steps of:
Step 1: placing the instrument 5 into the cleansing basin 101, sealing the cleansing basin 101, and simultaneously adding the cleansing water and the detergent to prepare a cleansing solution; subsequently, heating the cleansing solution by the cleansing basin heating pipe 103; initiating the ultrasonic sterilizing device 104, thereby cleansing and sterilizing the instrument 5 via ultrasonic wave; soaking the instrument 5 in the cleansing solution for a period of time;
Step 2: turning on the vacuum pump valve 110 and initiating the vacuum pump 111; subsequently, exhausting the air in the cleansing basin 101, thereby enabling the cleansing solution to be boiled under a low-temperature vacuum condition; cleansing the instrument 5; subsequently, turning off the vacuum pump valve 110 and the vacuum pump 111 after the vacuum environment is formed;
Step 3: turning on the bottom air inlet valve 202, and initiating the air filter 204 and the plasma sterilizer 203; allowing the air to enter into the cleansing basin 101 through the bottom air inlet coil pipe after being filtered and sterilized by the air filter 204 and the plasma sterilizer 203; enabling the cleansing water to boil vigorously to cleanse the instrument 5;
Step 4: turning on the vacuum pump valve 110 and initiating the vacuum pump 111; subsequently, exhausting the air in the cleansing basin 101, thereby enabling the cleansing solution to be boiled under a low-temperature vacuum condition; cleansing the instrument 5; subsequently, turning off the vacuum pump valve 110 and the vacuum pump 111 after the vacuum environment is formed;
Step 5: turning on the top portion air return valve 205, and allowing the air to enter into the cleansing basin 101 through the top portion air return valve 205 after being filtered and sterilized by the air filter 204 and the plasma sterilizer 203; enabling the pressure in the cleansing basin 101 to increase rapidly so that the steam in the steam coil 102 can be compressed and liquefied accordingly; allowing the cleansing water to flow into the steam coil 102, thereby cleansing and sterilizing the instrument 5;
Step 6: repeating the above steps 2-5 for several times, thereby removing the dirt; turning on the water discharge valve 105 to discharge the cleansing water; turning on the cleansing basin purified water inlet valve 305, thereby allowing the purified water in the purified water tank 306 to enter into the cleansing basin 101; heating the purified water by the cleansing basin heating pipe 101; repeating the above steps 2-5, and turning on the water discharge valve 105 to discharge purified water;
Step 7: turning on the water storage tank outlet valve 303, thereby allowing the purified water that is heated by the water storage tank heating pipe 302 in the water storage tank 301 to enter into the cleansing basin 101; keeping the purified water at a temperature of 93°C and sterilizing the instrument 5; after repeating the above steps 2-5, turning on the water discharge valve to discharge the heated purified water;
Step 8: initiating the vacuum pump to vacuum the cleansing basin 101, thereby forming a vacuum environment therein; after the steam in the instrument 5 is exhausted, turning on the steam valve 106 along the decrease of the vacuum degree, thereby allowing the steam to flow through the steam coil 102 so that the heat energy can be transferred through the aluminum plate 112 to heat the cleansing basin 101; when the temperature and the vacuum degree simultaneously increase, re-vacuuming by the vacuum pump 111, thereby forming a vacuum environment; after repeating the above steps for several times, turning on the top portion air return valve to achieve an air return, thereby making the air pressure in the cleansing basin 101 return to the atmospheric pressure; finally, turning off the ultrasonic sterilizing device 104, and taking out the sterilized instrument 5.

When the instrument 5 to be cleansed is a tubular structure, water steam is generated inside the tubular instrument, and the cleansing water is prevented from entering into the interior of the instrument under the negative pressure. When returning to the atmospheric pressure, water steam instantly turns into water, and the interior of the tubular instrument is flushed by the cleansing water. Thus, the instrument can be thoroughly cleansed, achieving an ideal cleansing effect.

Compared with the prior art, the present invention has the following advantages:
The cleansing and sterilizing device of the present invention uses a cleansing method combining a vacuum pulse and ultrasonic sterilization to perform cleansing and sterilization on medical instruments. The air purification device and the water purification device ensure that air and water entering into the sterilizing and cleansing device satisfy a cleansing requirement. The present invention replaces the traditional classified and manual cleansing by a fully automatic concentrated cleansing and sterilization, exhibits a favorable cleansing effect, achieves a high cleansing efficiency, and alleviates the labor burden of cleansing personnel.

It should be noted that the use of relational terms such as "first" and "second" are merely used to distinguish one entity or action from another entity or action without necessarily requiring or implying any actual relationship or order between such entities or actions. Furthermore, the terms "comprising" or "comprises", when used in this description and in the appended claims to indicate included features, elements or steps, is in no way to be interpreted as excluding the presence of other features, elements or steps than those expressly stated.

The description of above embodiments allows those skilled in the art to realize or use the present invention. Within the scope of the appended claims, those skilled in the art can combine, change or modify correspondingly according to the present invention. Therefore, the protective range of the present invention should not be limited to the embodiments above but conform to the widest protective range which is consistent with the scope of the appended claims.

## Claims

1. A sterilizing and cleansing device (1) using pulse vacuum, comprising:
a pipeline (4), a cleansing basin (101), a cleansing basin heating pipe (103), an ultrasonic sterilizing device (104), a water discharge valve (105), a manually-operated valve (107), a detergent valve (108), a detergent compartment (109), a vacuum pump valve (110), and a vacuum pump (111), wherein the cleansing basin (101) is a hollow cavity structure, and the bottom surface of the cleansing basin (101) is connected to the cleansing basin heating pipe (103) and the ultrasonic sterilizing device (104), wherein the cleansing basin (101) is connected to the water discharge valve (105) through the pipeline (4), and the lower portion of the cleansing basin (101) is connected to the detergent compartment (109) through the pipeline (4), wherein the detergent valve (108) is arranged on the pipeline (4) between the cleansing basin (101) and the detergent compartment (109), wherein the upper portion of the cleansing basin (101) is connected to the vacuum pump (111) through the pipeline (4), and the vacuum pump valve (110) is arranged on the pipeline (4) between the cleansing basin (101) and the vacuum pump (111), **characterized in that** said sterilizing and cleansing device (1) comprises:
an air purification device (2) and a water purification device (3) connected to the sterilizing and cleansing device (1),
a steam coil (102), a steam valve (106), and an aluminum plate (112), wherein the steam coil (102) is arranged in the interior of the side wall and the top portion of the cleansing basin (101), wherein the inner surface of the cleansing basin (101) is provided with the aluminum plate (112), and wherein the steam valve (106) and the manually-operated valve (107) are arranged on the steam coil (102).

2. The sterilizing and cleansing device (1) using pulse vacuum of claim 1, wherein the air purification device (2) further comprises a bottom air inlet coil pipe (201), a bottom air inlet valve (202), a plasma sterilizer (203), an air filter (204) and a top portion air return valve (205), wherein the bottom surface of the cleansing basin (101) is connected to the bottom air inlet coil pipe (201), and the bottom air inlet coil pipe (201) is connected to the plasma sterilizer (203) through the pipeline (4), wherein the bottom air inlet valve is arranged on the pipeline (4) between the bottom air inlet coil pipe (201) and the plasma sterilizer (203), wherein the plasma sterilizer (203) is connected to the air filter (204) and the top portion of the cleansing basin (101) through the pipeline (4), wherein the top portion air return valve (205) is arranged on the pipeline (4) between the plasma sterilizer (203) and the top portion of the cleansing basin (101).

3. The sterilizing and cleansing device (1) using pulse vacuum of claim 1, wherein the water purification device (3) further comprises a water storage tank (301), a water storage tank heating pipe (302), a water storage tank outlet valve (303), a water storage tank purified water inlet valve (304), a cleansing basin purified water inlet valve (305) and a purified water tank (306), wherein the lower portion of the cleansing basin (101) is connected to the water storage tank (301) and the purified water tank (306) through the pipeline (4), wherein the water storage tank outlet valve (303) is arranged on the pipeline (4) between the lower portion of the cleansing basin (101) and the water storage tank (301), wherein the cleansing basin purified water inlet valve 305 is arranged on the pipeline (4) between the lower portion of the water cleansing basin 101 and the purified water tank (306), wherein the water storage tank heating pipe (302) is arranged within the water storage tank (301), and the water storage tank (301) is connected to the purified water tank (306) through the pipeline (4), wherein the water storage tank purified water inlet valve (304) is arranged on the pipeline (4) between the water storage tank (301) and the purified water tank (306).

4. A cleansing method using the sterilizing and cleansing device (1) of claim 1, comprising the steps of:
Step 1: placing an instrument (5) into the cleansing basin (101), sealing the cleansing basin (101), and simultaneously adding the cleansing water and the detergent to prepare a cleansing solution; subsequently, heating the cleansing solution by the cleansing basin heating pipe (103); initiating the ultrasonic sterilizing device (104), thereby cleansing and sterilizing the instrument (5) via ultrasonic wave; soaking the instrument (5) in the cleansing solution for a period of time;
Step 2: turning on the vacuum pump valve (110) and initiating the vacuum pump (111); subsequently, exhausting the air in the cleansing basin (101), thereby enabling the cleansing solution to be boiled under a low-temperature vacuum condition; cleansing the instrument (5); subsequently, turning off the vacuum pump valve (110) and the vacuum pump (111) after the vacuum environment is formed;
Step 3: turning on a bottom air inlet valve (202), and initiating an air filter (204) and a plasma sterilizer (203); allowing the air to enter into the cleansing basin (101) through a bottom air inlet coil pipe after being filtered and sterilized by the air filter (204) and the plasma sterilizer (203); enabling the cleansing water to boil vigorously to cleanse the instrument (5);
Step 4: turning on the vacuum pump valve (110) and initiating the vacuum pump (111); subsequently, exhausting the air in the cleansing basin (101), thereby enabling the cleansing solution to be boiled under a low-temperature vacuum condition; cleansing the instrument (5); subsequently, turning off the vacuum pump valve (110) and the vacuum pump (111) after the vacuum environment is formed;
Step 5: turning on a top portion air return valve (205), and allowing the air to enter into the cleansing basin (101) through the top portion air return valve (205) after being filtered and sterilized by the air filter (204) and the plasma sterilizer (203); enabling the pressure in the cleansing basin (101) to increase rapidly so that the steam in the steam coil (102) can be compressed and liquefied accordingly; allowing the cleansing water to flow into the steam coil (102), thereby cleansing and sterilizing the instrument (5);
Step 6: repeating the above steps 2-5 for several times, thereby removing the dirt; turning on the water discharge valve (105) to discharge the cleansing water; turning on a cleansing basin purified water inlet valve (305), thereby allowing the purified water in a purified water tank (306) to enter into the cleansing basin (101); heating the purified water by the cleansing basin heating pipe (101); repeating the above steps 2-5, and turning on the water discharge valve (105) to discharge purified water;
Step 7: turning on a water storage tank outlet valve (303), thereby allowing the purified water that is heated by a water storage tank heating pipe (302) in a water storage tank (301) to enter into the cleansing basin (101); keeping the purified water at a temperature of 93°C and sterilizing the instrument (5); after repeating the above steps 2-5, turning on the water discharge valve (105) to discharge the heated purified water;
Step 8: initiating the vacuum pump to vacuum the cleansing basin (101), thereby forming a vacuum environment therein; after the steam in the instrument (5) is exhausted, turning on the steam valve (106) along the decrease of the vacuum degree, thereby allowing the steam to flow through the steam coil (102) so that the heat energy can be transferred through the aluminum plate (112) to heat the cleansing basin (101); when the temperature and the vacuum degree simultaneously increase, re-vacuuming by the vacuum pump (111), thereby forming a vacuum environment; after repeating the above steps for several times, turning on the top portion air return valve to achieve an air return, thereby making the air pressure in the cleansing basin (101) return to the atmospheric pressure; finally, turning off the ultrasonic sterilizing device (104), and taking out the sterilized instrument (5).

## Patentansprüche

1. Sterilisier- und Reinigungsvorrichtung (1), die Pulsvakuum verwendet, die Folgendes aufweist:
eine Rohrleitung (4), ein Reinigungsbecken (101), ein Reinigungsbeckenheizungsrohr (103), eine Ultraschallsterilisiervorrichtung (104), ein Wasserablassventil (105), ein manuell bedientes Ventil (107), ein Reinigungsmittelventil (108), einen Reinigungsmittelbehälter (109), ein Vakuumpumpenventil (110) und eine Vakuumpumpe (111), wobei das Reinigungsbecken (101) eine hohle Hohlraumkonstruktion ist und die Bodenfläche des Reinigungsbeckens (101) mit dem Reinigungsbeckenheizungsrohr (103) und der Ultraschallsterilisiervorrichtung (104) verbunden ist, wobei das Reinigungsbecken (101) über die Rohrleitung (4) mit dem Wasserablassventil (105) verbunden ist und der untere Abschnitt des Reinigungsbeckens (101) über die Rohrleitung (4) mit dem Reinigungsmittelbehälter (109) verbunden ist, wobei das Reinigungsmittelventil (108) an der Rohrleitung (4) zwischen dem Reinigungsbecken (101) und dem Reinigungsmittelbehälter (109) angeordnet ist, wobei der obere Abschnitt des Reinigungsbeckens (101) über die Rohrleitung (4) mit der Vakuumpumpe (111) verbunden ist und das Vakuumpumpenventil (110) an der Rohrleitung (4) zwischen dem Reinigungsbecken (101) und der Vakuumpumpe (111) angeordnet ist, **dadurch gekennzeichnet, dass** die Sterilisier- und Reinigungsvorrichtung (1) Folgendes aufweist:
eine Luftreinigungsvorrichtung (2) und eine Wasserreinigungsvorrichtung (3), die mit der Sterilisier- und Reinigungsvorrichtung (1) verbunden sind, eine Dampfschlange (102), ein Dampfventil (106) und eine Aluminiumplatte (112), wobei die Dampfschlange (102) innerhalb der Seitenwand und des oberen Teils des Reinigungsbeckens (101) angeordnet ist, wobei die Innenfläche des Reinigungsbeckens (101) mit der Aluminiumplatte (112) versehen ist, und wobei das Dampfventil (106) und das manuell bediente Ventil (107) an der Dampfschlange (102) angeordnet sind.

2. Sterilisier- und Reinigungsvorrichtung (1), die Pulsvakuum verwendet, nach Anspruch 1, wobei die Luftreinigungsvorrichtung (2) ferner eine untere Lufteinlassrohrschlange (201), ein unteres Lufteinlassventil (202), einen Plasmasterilisator (203), ein Luftfilter (204) und ein Luftrücklaufventil (205) des oberen Teils aufweist, wobei die Bodenfläche des Reinigungsbeckens (101) mit der unteren Lufteinlassrohrschlange (201) verbunden ist und die untere Lufteinlassrohrschlange (201) über die Rohrleitung (4) mit dem Plasmasterilisator (203) verbunden ist, wobei das untere Lufteinlassventil an der Rohrleitung (4) zwischen der unteren Lufteinlassrohrschlange (201) und dem Plasmasterilisator (203) angeordnet ist, wobei der Plasmasterilisator (203) über die Rohrleitung (4) mit dem Luftfilter (204) und dem oberen Teil des Reinigungsbeckens (101) verbunden ist, wobei das Luftrückfuhrventil (205) des oberen Teils an der Rohrleitung (4) zwischen dem Plasmasterilisator (203) und dem oberen Teil des Reinigungsbeckens (101) angeordnet ist.

3. Sterilisier- und Reinigungsvorrichtung (1), die Pulsvakuum verwendet, nach Anspruch 1, wobei die Wasserreinigungsvorrichtung (3) ferner einen Wasserspeichertank (301), ein Wasserspeichertankheizungsrohr (302), ein Wasserspeichertankauslassventil (303), ein Wasserspeichertrankeinlassventil (304) für gereinigtes Wasser, ein Reinigungsbeckeneinlassventil (305) für gereinigtes Wasser und einen Tank (306) für gereinigtes Wasser aufweist, wobei der untere Abschnitt des Reinigungsbeckens (101) über die Rohrleitung (4) mit dem Wasserspeichertank (301) und dem Tank (306) für gereinigtes Wasser verbunden ist, wobei das Wasserspeichertankauslassventil (303) an der Rohrleitung (4) zwischen dem untere Abschnitt des Reinigungsbeckens (101) und dem Wasserspeichertank (301) angeordnet ist, wobei das Reinigungsbeckeneinlassventil (305) für gereinigtes Wasser an der Rohrleitung (4) zwischen dem unteren Abschnitt des Wasserreinigungsbeckens (101) und dem Tank (306) für gereinigtes Wasser angeordnet ist, wobei das Wasserspeichertankheizungsrohr (302) innerhalb des Wasserspeichertanks (301) angeordnet ist, und wobei der Wasserspeichertank (301) über die Rohrleitung (4) mit dem Tank (306) für gereinigtes Wasser verbunden ist, wobei das Wasserspeichertankeinlassventil (304) für gereinigtes Wasser an der Rohrleitung (4) zwischen dem Wasserspeichertank (301) und dem Tank (306) für gereinigtes Wasser angeordnet ist.

4. Reinigungsverfahren unter Verwendung der Sterilisier- und Reinigungsvorrichtung (1) nach Anspruch 1, das die folgenden Schritte aufweist:
Schritt 1: Platzieren eines Instruments (5) in dem Reinigungsbecken (101), Verschließen des Reinigungsbeckens (101) und gleichzeitig Hinzugeben des Reinigungswassers und des Reinigungsmittels, um eine Reinigungslösung zu bereiten; nachfolgend Erwärmen der Reinigungslösung mittels des Reinigungsbeckenheizungsrohrs (103); Starten der Ultraschallsterilisiervorrichtung (104), dadurch Reinigen und Sterilisieren des Instruments (5) mittels Ultraschallwelle; Einweichen des Instruments (5) in der Reinigungslösung für eine Zeitdauer;
Schritt 2: Einschalten des Vakuumpumpenventils (110) und Starten der Vakuumpumpe (111); nachfolgend Ablassen der Luft in dem Reinigungsbecken (101), wodurch ermöglicht wird, dass die Reinigungslösung unter Vakuumbedingungen bei niedriger Temperatur siedet; Reinigen des Instruments (5); nachfolgend Ausschalten des Vakuumpumpenventils (110) und der Vakuumpumpe (111), nachdem die Vakuumumgebung gebildet ist;
Schritt 3: Einschalten eines unteren Lufteinlassventils (202) und Starten eines Luftfilters (204) eines Plasmasterilisators (203); Gestatten, dass die Luft durch die untere Lufteinlassrohrschlange in das Reinigungsbecken (101) eintritt, nachdem sie von dem Luftfilter (204) und dem Plasmasterilisator (203) gefiltert und sterilisiert wurde; Ermöglichen, dass das Reinigungswasser stark siedet, um das Instrument (5) zu reinigen;
Schritt 4: Einschalten des Vakuumpumpenventils (110) und Starten der Vakuumpumpe (111); nachfolgend Ablassen der Luft in dem Reinigungsbecken (101), wodurch ermöglicht wird, dass die Reinigungslösung unter Vakuumbedingungen bei niedriger Temperatur siedet; Reinigen des Instruments (5); nachfolgend Ausschalten des Vakuumpumpenventils (110) und der Vakuumpumpe (111), nachdem die Vakuumumgebung gebildet ist;
Schritt 5: Einschalten eines Luftrückfuhrventils (205) des oberen Teils und Gestatten, dass Luft durch das Luftrückfuhrventils (205) des oberen Teils in das Reinigungsbecken (101) eintritt, nachdem sie von dem Luftfilter (204) und dem Plasmasterilisator (203) gefiltert und sterilisiert wurde; Ermöglichen, dass der Druck in dem Reinigungsbecken (101) schnell zunimmt, damit der Dampf in der Dampfschlange (102) entsprechende komprimiert und verflüssigt werden kann; Gestatten, dass das Reinigungswasser in die Dampfschlange (102) fließt, wodurch das Instrument (5) gereinigt und sterilisiert wird;
Schritt 6: Mehrmaliges Wiederholen der obigen Schritte 2 bis 5, damit Entfernen von Schmutz; Einschalten des Wasserablassventils (105), um das Reinigungswasser abzulassen; Einschalten des Reinigungsbeckeneinlassventils (305) für gereinigtes Wasser, dadurch Gestatten, dass gereinigtes Wasser in einem Tank (306) für gereinigtes Wasser in das Reinigungsbecken (101) eintritt; Erwärmen des gereinigten Wassers mittels des Reinigungsbeckenheizungsrohrs (101); Wiederholen der obigen Schritte 2 bis 5 und Einschalten des Wasserauslassventils (105), um gereinigtes Wasser auszulassen;
Schritt 7: Einschalten eines Wasserspeichertankauslassventils (303), dadurch Gestatten, dass das gereinigte Wasser, das durch ein Wasserspeichertankheizungsrohr (302) in einem Wasserspeichertank (301) erwärmt wird, in das Reinigungsbecken (101) eintritt; Halten des gereinigten Wassers auf einer Temperatur von 93°C und Sterilisieren des Instruments (5); nach Wiederholen der obigen Schritte 2 bis 5, Einschalten des Wasserablassventils (105), um das erwärmte gereinigte Wasser abzulassen;
Schritt 8: Starten der Vakuumpumpe, um das Reinigungsbecken (101) abzusaugen, wodurch eine Vakuumumgebung darin gebildet wird; nachdem der Dampf in dem Instrument (5) abgelassen ist, Einschalten des Dampfventils (106) entlang des Abfalls des Vakuumwerts, dadurch Gestatten, dass der Dampf durch die Dampfschlange (102) fließt, derart dass die Wärmeenergie über die Aluminiumplatte (112) zum Erwärmen des Reinigungsbeckens (101) übertragen werden kann; wenn sich die Temperatur und der Vakuumwert gleichzeitig erhöhen, erneutes Absaugen mittels der Vakuumpumpe (111), dadurch Bilden einer Vakuumumgebung; nach mehrmaligem Wiederholen der obigen Schritte, Einschalten des Luftrückfuhrventils des oberen Teils, um eine Luftrückfuhr zu erzielen, dadurch Herbeiführen, dass der Luftdruck in dem Reinigungsbecken (101) auf den Umgebungsluftdruck zurückkehrt; Schließlich Ausschalten der Ultraschallsterilisiervorrichtung (104) und Entnehmen des sterilisierten Instruments (5).

## Revendications

1. Dispositif de nettoyage et de stérilisation (1) utilisant un vide à impulsions, comprenant :
une tuyauterie (4), une cuvette de nettoyage (101), un tuyau de chauffage de cuvette de nettoyage (103), un dispositif de stérilisation ultrasonore (104), une soupape d'évacuation d'eau (105), une soupape actionnée manuellement (107), une soupape de détergent (108), un compartiment de détergent (109), une soupape de pompe à vide (110) et une pompe à vide (111), dans lequel la cuvette de nettoyage (101) est une structure à cavité creuse et la surface inférieure de la cuvette de nettoyage (101) est raccordée au tuyau de chauffage de cuvette de nettoyage (103) et au dispositif de stérilisation ultrasonore (104), dans lequel la cuvette de nettoyage (101) est raccordée à la soupape d'évacuation d'eau (105) via la tuyauterie (4) et la partie inférieure de la cuvette de nettoyage (101) est raccordée au compartiment de détergent (109) via la tuyauterie (4), dans lequel la soupape de détergent (108) est agencée sur la tuyauterie (4) entre la cuvette de nettoyage (101) et le compartiment de détergent (109), dans lequel la partie supérieure de la cuvette de nettoyage (101) est raccordée à la pompe à vide (111) via la tuyauterie (4) et la soupape de pompe à vide (110) est agencée sur la tuyauterie (4) entre la cuvette de nettoyage (101) et la pompe à vide (111), **caractérisé en ce que** ledit dispositif de nettoyage et de stérilisation (1) comprend :
un dispositif de purification d'air (2) et un dispositif de purification d'eau (3) raccordés au dispositif de nettoyage et de stérilisation (1), un serpentin de vapeur (102), une soupape de prise de vapeur (106) et une plaque d'aluminium (112), dans lequel le serpentin de vapeur (102) est disposé à l'intérieur de la paroi latérale et la partie supérieure de la cuvette de nettoyage (101), dans lequel la surface intérieure de la cuvette de nettoyage (101) est dotée de la plaque d'aluminium (112), et dans lequel la soupape de prise de vapeur (106) et la soupape actionnée manuellement (107) sont agencées sur le serpentin de vapeur (102).

2. Dispositif de nettoyage et de stérilisation (1) utilisant un vide à impulsions selon la revendication 1, dans lequel le dispositif de purification d'air (2) comprend en outre un serpentin d'admission d'air inférieur (201), une soupape d'admission d'air inférieure (202), un stérilisateur à plasma (203), un filtre à air (204) et une soupape de retour d'air de partie supérieure (205), dans lequel la surface inférieure de la cuvette de nettoyage (101) est raccordée au serpentin d'admission d'air inférieur (201) et le serpentin d'admission d'air inférieur (201) est raccordé au stérilisateur à plasma (203) via la tuyauterie (4), dans lequel la soupape d'admission d'air inférieure est disposée sur la tuyauterie (4) entre le serpentin d'admission d'air inférieur (201) et le stérilisateur à plasma (203), dans lequel le stérilisateur à plasma (203) est raccordé au filtre à air (204) et à la partie supérieure de la cuvette de nettoyage (101) via la tuyauterie (4), dans lequel la soupape de retour d'air de partie supérieure (205) est agencée sur la tuyauterie (4) entre le stérilisateur à plasma (203) et la partie supérieure de la cuvette de nettoyage (101).

3. Dispositif de nettoyage et de stérilisation (1) utilisant un vide à impulsions selon la revendication 1, dans lequel le dispositif de purification d'eau (3) comprend en outre un réservoir de stockage d'eau(301), un tuyau de chauffage de réservoir de stockage d'eau (302), une soupape de sortie de réservoir de stockage d'eau (303), une soupape d'entrée d'eau purifiée de réservoir de stockage d'eau (304), une soupape d'entrée d'eau purifiée de cuvette de nettoyage (305) et un réservoir d'eau purifiée (306), dans lequel la partie inférieure de la cuvette de nettoyage (101) est raccordée au réservoir de stockage d'eau (301) et au réservoir d'eau purifiée (306) via la tuyauterie (4), dans lequel la soupape de sortie de réservoir de stockage d'eau (303) est agencée sur la tuyauterie (4) entre la partie inférieure de la cuvette de nettoyage (101) et le réservoir de stockage d'eau (301), dans lequel la soupape d'entrée d'eau purifiée de cuvette de nettoyage (305) est agencée sur la tuyauterie (4) entre la partie inférieure de la cuvette de nettoyage d'eau (101) et le réservoir d'eau purifiée (306), dans lequel le tuyau de chauffage de réservoir de stockage d'eau (302) est disposé dans le réservoir de stockage d'eau (301), et le réservoir de stockage d'eau (301) est raccordé au réservoir d'eau purifiée (306) via la tuyauterie (4), dans lequel la soupape d'entrée d'eau purifiée de réservoir de stockage d'eau (304) est agencée sur la tuyauterie (4) entre le réservoir de stockage d'eau (301) et le réservoir d'eau purifiée (306).

4. Procédé de nettoyage utilisant le dispositif de nettoyage et de stérilisation (1) selon la revendication 1, comprenant les étapes suivantes :
étape 1 : placer un instrument (5) dans la cuvette de nettoyage (101), fermer la cuvette de nettoyage (101) de manière étanche et ajouter simultanément de l'eau de nettoyage et du détergent pour préparer une solution de nettoyage ; de manière subséquente, chauffer la solution de nettoyage grâce au tuyau de chauffage de cuvette de nettoyage (103) ; démarrer le dispositif de stérilisation ultrasonore (104) en nettoyant et stérilisant ainsi l'instrument (5) via des ondes ultrasonores ; faire tremper l'instrument (5) dans la solution de nettoyage pendant une période de temps ;
étape 2 : mettre en marche de la soupape de pompe à vide (110) et démarrer la pompe à vide (111) ; de manière subséquente, faire s'échapper l'air dans la cuvette de nettoyage (101) en permettant ainsi de porter à ébullition la solution de nettoyage dans des conditions sous vide à basse température ; nettoyer l'instrument (5) ; de manière subséquente, arrêter la soupape de pompe à vide (110) et la pompe à vide (111) une fois l'environnement sous vide formé ;
étape 3 : mettre en marche une soupape d'admission d'air inférieure (202) et démarrer un filtre à air (204) et un stérilisateur à plasma (203) ; permettre à l'air d'entrer dans la cuvette de nettoyage (101) via un serpentin d'admission d'air inférieur après avoir été filtré et stérilisé par le filtre à air (204) et le stérilisateur à plasma (203) ; permettre à l'eau de nettoyage de bouillir vigoureusement pour nettoyer l'instrument (5) ;
étape 4 : mettre en marche la soupape de pompe à vide (110) et démarrer la pompe à vide (111) ; de manière subséquente, faire s'échapper l'air dans la cuvette de nettoyage (101) en permettant ainsi à la solution de nettoyage d'être portée à ébullition dans des conditions sous vide à basse température ; nettoyer l'instrument (5) ; de manière subséquente, arrêter la soupape de pompe à vide (110) et la pompe à vide (111) une fois que l'environnement sous vide est formé ;
étape 5 : mettre en marche une soupape de retour d'air de partie supérieure (205) et permettre à l'air d'entrer dans la cuvette de nettoyage (101) via la soupape de retour d'air de partie supérieure (205) après avoir été filtré et stérilisé par le filtre à air (204) et le stérilisateur à plasma (203) ; permettre à la pression dans la cuvette de nettoyage (101) d'augmenter rapidement de sorte que la vapeur dans le serpentin de vapeur (102) puisse être comprimée et liquéfiée de manière conséquente ; permettre à l'eau de nettoyage de s'écouler dans le serpentin de vapeur (102) en nettoyant et stérilisant ainsi l'instrument (5) ;
étape 6 : répéter plusieurs fois les étapes 2-5 ci-dessus en éliminant ainsi la saleté ; mettre en marche la soupape d'évacuation d'eau (105) pour évacuer l'eau de nettoyage ; mettre en marche une soupape d'entrée d'eau purifiée de cuvette de nettoyage (305) en permettant ainsi à l'eau purifiée dans le réservoir d'eau purifiée (306) d'entrer dans la cuvette de nettoyage (101); chauffer l'eau purifiée grâce au tuyau de chauffage de cuvette de nettoyage (101) ; répéter les étapes 2-5 ci-dessus et mettre en marche la soupape d'évacuation d'eau (105) pour évacuer l'eau purifiée ;
étape 7 : mettre en marche une soupape de sortie de réservoir de stockage d'eau (303) en permettant ainsi à l'eau purifiée qui est chauffée par un tuyau de chauffage de réservoir de stockage d'eau (302) dans un réservoir de stockage d'eau (301) d'entrer dans la cuvette de nettoyage (101); maintenir l'eau purifiée à une température de 93°C et stériliser l'instrument (5) ; après avoir répété les étapes 2-5 ci-dessus, mettre en marche la soupape d'évacuation d'eau (105) pour évacuer l'eau purifiée chauffée ;
étape 8 : démarrer la pompe à vide pour mettre la cuvette de nettoyage (101) sous vide en formant ainsi un environnement sous vide dans celle-ci ; une fois que la vapeur dans l'instrument (5) s'est échappée, mettre en marche la soupape de prise de vapeur (106) tout au long de la diminution du degré de vide, en permettant ainsi à la vapeur de s'écouler à travers le serpentin de vapeur (102) de sorte que l'énergie de chaleur peut être transférée via la plaque d'aluminium (112) pour chauffer la cuvette de nettoyage (101) ; lorsque la température et le degré de vide augmentent simultanément, remettre sous vide grâce à la pompe à vide (111) en formant ainsi un environnement sous vide ; après avoir répété plusieurs fois les étapes ci-dessus, mettre en marche la soupape de retour d'air de partie supérieure pour obtenir un retour d'air en faisant ainsi revenir la pression d'air dans la cuvette de nettoyage (101) à la pression atmosphérique ; finalement, arrêter le dispositif de stérilisation ultrasonore (104) et retirer l'instrument stérilisé (5).
